(19) Europäisches Patentamt / European Patent Office / Office européen des brevets

(11) **EP 3 151 812 B1**

(12) **EUROPEAN PATENT SPECIFICATION**

(45) Date of publication and mention
of the grant of the patent:
**22.04.2020  Bulletin 2020/17**

(51) Int Cl.:
*A61K 9/00* *(2006.01)*  *A61K 31/167* *(2006.01)*
*A61K 31/40* *(2006.01)*  *A61K 31/573* *(2006.01)*

(21) Application number: **15731264.6**

(22) Date of filing: **09.06.2015**

(86) International application number:
**PCT/EP2015/062764**

(87) International publication number:
**WO 2015/189168 (17.12.2015 Gazette 2015/50)**

(54) **INHALATION PARTICLES COMPRISING A COMBINATION OF AN ANTICHOLINERGIC, A CORTICOSTEROID AND A BETA-ADRENERGIC**

INHALATIONSPARTIKEL MIT EINER KOMBINATION AUS EINEM ANTICHOLINERGIKUM, EINEM CORTICOSTEROID UND EINEM BETA-ADRENERGIKUM

PARTICULES D'INHALATION COMPRENANT UNE COMBINAISON D'UN ANTICHOLINERGIQUE, UN CORTICOSTÉROÏDE ET UN BÊTA-ADRÉNERGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **09.06.2014  EP 14171644**

(43) Date of publication of application:
**12.04.2017  Bulletin 2017/15**

(73) Proprietor: **Chiesi Farmaceutici S.p.A.**
**43100 Parma (IT)**

(72) Inventors:
• **MIOZZI, Michele**
**I-43100 Parma (IT)**
• **ROUSE, Timothy J**
**I-43100 Parma (IT)**
• **BRAMBILLA, Gaetano**
**I-43100 Parma (IT)**

(74) Representative: **Bianchetti Bracco Minoja S.r.l.**
**Via Plinio, 63**
**20129 Milano (IT)**

(56) References cited:
**US-A1- 2011 150 782     US-A1- 2011 262 543**

• **M. CAZZOLA ET AL: "Long-acting bronchodilators in COPD: where are we now and where are we going?", BREATHE, vol. 10, no. 2, 1 June 2014 (2014-06-01), pages 110-120, XP055134705, ISSN: 1810-6838, DOI: 10.1183/20734735.014813**

**Description**

**TECHNICAL FIELD**

The present invention relates to particles comprising three active ingredients for administration by inhalation.

In particular, the invention relates to particles comprising a combination of an anticholinergic, a beta$_2$-adrenoceptor agonist, and an inhaled corticosteroid, process for their preparation and use thereof for the prevention and/or treatment of respiratory diseases.

**BACKGROUND OF THE INVENTION**

Respiratory diseases are a common and important cause of illness and death around the world. In fact, many people are affected by inflammatory and/or obstructive lung diseases, a category characterized by inflamed and easily collapsible airways, obstruction to airflow, problems exhaling and frequent medical clinic visits and hospitalizations. Types of inflammatory and/or obstructive lung disease include asthma, bronchiectasis, bronchitis and chronic obstructive pulmonary disease (COPD).

In particular, chronic obstructive pulmonary disease (COPD) is a multi-component disease characterized by airflow limitation and airway inflammation. Exacerbations of COPD have a considerable impact on the quality of life, daily activities and general well-being of patients and are a great burden on the health system. Thus, the aims of COPD management include not only relieving symptoms and preventing disease progression but also preventing and treating exacerbations.

While available therapies improve clinical symptoms and decrease airway inflammation, they do not unequivocally slow long-term progression or address all disease components. With the burden of COPD continuing to increase, research into new and improved treatment strategies to optimize pharmacotherapy is ongoing, and in particular, combination therapies, with a view to their complementary modes of action enabling multiple components of the disease to be addressed. Evidence from recent clinical trials indicates that triple therapy, combining an anticholinergic with an inhaled corticosteroid, and a long-acting $\beta_2$-adrenoceptor agonist, may provide clinical benefits additional to those associated with each treatment alone in patients with more severe COPD.

Currently, there are several recommended classes of therapy for COPD, of which bronchodilators such as $\beta_2$-agonists and anticholinergics are the mainstay of symptom management in mild and moderate disease, prescribed on an as-needed basis for mild COPD and as a maintenance therapy for moderate COPD.

Said bronchodilators are efficiently administered by inhalation, thus increasing the therapeutic index and reducing side effects of the active material.

For the treatment of more severe COPD, guidelines recommend the addition of inhaled corticosteroids (ICSs) to long-acting bronchodilator therapy. Combinations of therapies have been investigated with a view to their complementary modes of action enabling multiple components of the disease to be addressed. Data from recent clinical trials indicate that triple therapy, combining an anticholinergic with an ICS and a long-acting $\beta_2$-agonist (LABA), may provide clinical benefits additional to those associated with each treatment alone in patients with moderate to severe COPD.

Furthermore, there is evidence suggesting synergistic actions of the LABA and ICS as long as both active ingredients are present at the same site of action, for example the small peripheral airways of the pulmonary tree. Without being limited by the theory, this might also occur if the anti-muscarinic drug is delivered at said site of action.

An interesting triple combination, presently under investigation, includes:

i) formoterol, particularly its dihydrate fumarate salt (hereinafter indicated as FF), a long acting beta-2 adrenergic receptor agonist, currently used clinically in the treatment of bronchial asthma, COPD and related disorders;
ii) rac-glycopyrronium bromide (hereinafter indicated as GB), an antimuscarinic drug recently approved for the maintenance treatment of COPD;
iii) beclometasone dipropionate (hereinafter indicated as BDP) a potent anti-inflammatory corticosteroid steroid, available under a wide number of brands for the prophylaxis and/or treatment of asthma and other respiratory disorders.

On the other hand, current combined inhalation products may be subjected to a great variability in the dose delivery of each active ingredient, which in turn may be perpetuated as a function of product storage conditions.

Hence, it would be advantageous to provide a process enabling the preparation of combination-particles for inhalation that will allow all the three active ingredients to be delivered without significant dose variations.

Furthermore, there is still a need of an improved therapeutic control of individuals exhibiting respiratory diseases affecting the small peripheral airways.

Therefore it would highly be advantageous to provide a process enabling the preparation of combination-particles whereby all the three active ingredients could simultaneously reach the distal tract of the respiratory tree and hence improving small airways outcomes and associated control.

In the prior art, different approaches have been proposed for preparing particles incorporating a combination of two or more active ingredients. For example, WO 02/28377, WO 2010/097188, and WO 2013/021199 disclose particles incorporating, *inter alia,* a LABA and an ICS.

US 2011/150782 discloses aerosol formulations comprising glycopyrronium bromide in combination with formoterol useful for the prevention or treatment of chronic obstructive pulmonary disease. The formulation further comprises a HFA propellant, a co-solvent, and an amount of inorganic acid sufficient to stabilize both the glycopyrronium bromide and the formoterol components. Optionally the formulation may further comprise beclometasone dipropionate.

US 2011/262543 discloses a process for preparing carrier particles for a dry powder formulation for inhalation, in particular having reduced electrostatic charges, said carrier particles comprising: (i) a fraction of co-micronized particles made of a mixture of an excipient and an additive, the mixture having a mass median diameter (MMD) lower than 20 microns; and (ii) a fraction of coarse excipient particles having a MMD equal to or higher than 80 microns.

However, none of said documents disclose particles incorporating a combination of formoterol salts, beclometasone dipropionate and glycopyrronium salts. Furthermore, they are all silent about the problem of reaching the distal tract of the respiratory tree.

## SUMMARY OF THE INVENTION

The invention is directed to multicomponent microparticles for use in a formulation for inhalation, each microparticle comprising a combination of beclometasone dipropionate, formoterol fumarate, and a glycopyrronium bromide in a ratio comprised between 35:10:55 and 94:1:5 w/w/w, whereby said microparticles are characterized by a shape factor comprised between 0.95 and 1.05 and are obtainable by a process comprising the steps of:

a) preparing a solution of the three active ingredients in a pre-determined ratio in a solvent consisting of a mixture of ethanol:water in a ratio of from 85:15 to 95:5 v/v;
b) generating an aerosol from the solution of said three active ingredients and
c) drying the atomized droplets to yield the microparticles.

Advantageously at least 90% of all the above microparticles have a volume diameter equal to or lower than 4.5 micron, preferably equal to or lower than 4.0 micron, and the volume median diameter of said microparticles is comprised between 1.0 and 3.0 micron, preferably 1.2 and 2.5 micron, more preferably between 1.5 and 2.2 micron.

In a second aspect, the invention provides pharmaceutical aerosol formulations for pressurized metered dose inhalers (pMDIs) comprising the above microparticles in suspension in a liquefied propellant gas.

In a third aspect, the invention provides a pressurized metered dose inhaler (pMDI) comprising a canister filled with the aforementioned pharmaceutical aerosol formulation, and a metering valve for delivering a daily therapeutically effective dose of the active ingredient.

In a fourth aspect, the invention concerns a dry powder pharmaceutical formulation comprising the above microparticles and, optionally a carrier.

In a fifth aspect, the invention provides a dry powder inhaler filled with the aforementioned dry powder formulation.

In a sixth aspect, the invention is directed to a process for preparing the claimed microparticles, the process comprising the steps of:

a) preparing a solution of the three active ingredients in a pre-determined ratio in a suitable solvent;
b) generating an aerosol from the solution of said three active ingredients;
c) drying the atomized droplets to yield the microparticles; and
d) isolating the produced microparticles.

In a seventh aspect, the invention refers to the claimed microparticles for use in the prevention and/or treatment of an inflammatory and/or obstructive airways disease such as asthma or chronic obstructive pulmonary disease (COPD).

## DEFINITIONS

The terms "muscarinic receptor antagonists", "antimuscarinic drugs" and "anticholinergic drugs" can be used as synonymous.

The term "pharmaceutically acceptable salt of glycopyrronium" refers to a salt of the compound 3-[(cyclopentylhydroxyphenylacetyl)oxy]-1,1-dimethylpyrrolidinium.

The term "pharmaceutically acceptable salt of formoterol" refers to a salt of the compound 2'-hydroxy-5'-[(RS)-1-hydroxy-2-{[(RS)-p-methoxy-$\alpha$-methylphenethyl] amino} ethyl] formanilide.

The term "beclometasone dipropionate" refers to the compound (8$S$,9$R$,10$S$,11$S$,13$S$,14$S$,16$S$,17$R$)-9-chloro-11-hydroxy-10,13,16-trimethyl-3-oxo-17-[2-(propionyloxy)acetyl]-6,7,8,9,10,11,12,13,14,15,16,17-dodecahydro-3$H$-cyclopenta[$a$]phenanthren-17-ylpropionate.

The term "pharmaceutically acceptable salt" comprises inorganic and organic salts. Examples of organic salts may include formate, acetate, trifluoroacetate, propionate, butyrate, lactate, citrate, tartrate, malate, maleate, succinate, methane sulfonate, benzenesulfonate, xinafoate, pamoate, and benzoate. Examples of inorganic salts may include fluoride chloride, bromide, iodide, phosphate, nitrate and sulphate.

The term "solvent" is used to mean the medium in which the active ingredients are dissolved, while the term "anti-solvent" is used to mean the medium in which crystallization takes place.

The term "multicomponent particle" refers to the smallest discrete single particle comprising a combination of three active ingredients. Said single particle is engineered in spherical form.

The term "micronized" refers to a substance having a size of few microns.

The term "coarse" refers to a substance having a size of one or few hundred microns.

In general terms, the particle size of particles is quantified by measuring a characteristic equivalent sphere diameter, known as volume diameter, by laser diffraction.

The particle size can also be quantified by measuring the mass diameter by means of suitable known instrument such as, for instance, the sieve analyser.

The volume diameter (VD) is related to the mass diameter (MD) by the density of the particles (assuming a size independent density for the particles).

In the present application, the particle size of the active ingredients and of fraction of fine particles is expressed in terms of volume diameter, while that of the coarse particles is expressed in terms of mass diameter.

The particles have a normal (Gaussian) distribution which is defined in terms of the volume or mass median diameter (VMD or MMD) which corresponds to the volume or mass diameter of 50 percent by weight of the particles, and, optionally, in terms of volume or mass diameter of 10% and 90% of the particles, respectively.

Another common approach to define the particle size distribution is to cite three values: i) the median diameter d(0.5) which is the diameter where 50% of the distribution is above and 50% is below; ii) d(0.9), where 90% of the distribution is below this value; iii) d(0.1), where 10% of the distribution is below this value.

The span is the width of the distribution based on the 10%, 50% and 90% quantile and is calculated according to the formula.

$$\text{Span} = \frac{D[v,0.9] - D[v,0.1]}{D[v,0.5]}$$

In general terms, particles having the same or a similar VMD or MMD can have a different particle size distribution, and in particular a different width of the Gaussian distribution as represented by the d(0.1) and d(0.9) values.

Upon aerosolisation, the particle size is expressed as mass aerodynamic diameter (MAD), while the particle size distribution is expressed in terms of mass median aerodynamic diameter (MMAD) and Geometric Standard Deviation (GSD). The MAD indicates the capability of the particles of being transported suspended in an air stream. The MMAD corresponds to the mass aerodynamic diameter of 50 percent by weight of the particles.

In the final formulation, the particle size of the microparticles of the invention can be determined by scanning electron microscopy according to methods known to the skilled person in the art.

The term 'synergistic' means that the activity of the active ingredients is more than would be expected by summing their respective individual activities in a given assay.

The term 'interactive or ordered mixture' refers to powder formulation for inhalation comprising a pharmacologically-inert physiologically acceptable carrier substance, to which the micronised active compound particles are bonded by adhesion in order thus to achieve and to maintain a suitable mixed material, i.e. homogeneity of the mixture.

The term 'relatively highly fissured surface' means a surface on which there are clefts and valleys and other recessed regions, referred to herein collectively as fissures. Said surface of the coarse excipient particles may be defined in terms of fissure index or rugosity coefficients as disclosed in WO 01/78695 and WO 01/78693 and they can be characterized according to the description therein reported.

The term 'hard pellets' refers to spherical or semispherical units whose core is made of coarse excipient particles.

The term 'good flowability' refers to a formulation that is easy handled during the manufacturing process and is able to ensure an accurate and reproducible delivering of the therapeutically effective dose.

Flow characteristics can be evaluated by different tests such as angle of repose, Carr's index, Hausner ratio or flow rate through an orifice.

In the context of the present application the flow properties were tested by measuring the flow rate through an orifice according to the method described in the European Pharmacopeia (Eur. Ph.) 7.3, 7th Edition.

The expression 'good homogeneity' refers to a powder wherein, upon mixing, the uniformity of distribution of a component, expressed as coefficient of variation (CV) also known as relative standard deviation (RSD), is less than 5.0%. It is usually determined according to known methods, for instance by taking samples from different parts of the powder and testing the component by HPLC or other equivalent analytical methods such as UPLC.

The expression 'respirable fraction' refers to an index of the percentage of the active ingredient particles which would reach the lungs in a patient.

The respirable fraction is evaluated using a suitable *in vitro* apparatus such as Andersen Cascade Impactor (ACI), Multi Stage Liquid Impinger (MLSI) or Next Generation Impactor (NGI), according to procedures reported in common Pharmacopoeias, in particular in the European Pharmacopeia (Eur. Ph.) 7.3, 7th Edition.

It is calculated by the percentage ratio of the fine particle mass (formerly fine particle dose) to the delivered dose.

The delivered dose is calculated from the cumulative deposition in the apparatus, while the fine particle mass is calculated from the deposition of particles having a diameter < 5.0 micron.

In the context of the present application, the formulation is defined as extrafine formulation when it is able of delivering a fraction of particles having a particle size equal or less than 2.0 micron equal to or higher than 20%, preferably equal to or higher than 25%, more preferably equal to or higher than 30% and/or it is able of delivering a fraction of particles having a particle size equal or less than 1.0 micron equal to or higher than 10%, preferably equal to or higher than 20%.

The expression 'chemically stable' refers to an active ingredient that, upon storage of the microparticles, meets the requirements of the EMEA Guideline CPMP/QWP/122/02 referring to 'Stability Testing of Existing Active Substances and Related Finished Products'.

The expression 'physically stable' refers to microparticles which exhibit substantially no change in morphology, no transition from amorphous to crystalline state or *vice versa,* no growth in particle size during storage for at least one month at room temperature and 60% relative humidity, as determined according to methods known to the skilled person in the art.

The expression 'good constancy of the active ingredients ratio' means that the three active ingredients, after delivery of a single therapeutic dose, maintain substantially the same ratio as the pre-determined ratio of said two active ingredient in the formulation, i.e. that the relative standard deviation (RSD) of the ratio of the amounts of drugs measured in an vitro apparatus such as NGI is less than 15%, preferably less than 10%.

The term 'prevention' means an approach for reducing the risk of onset of a disease.

The term 'treatment' means an approach for obtaining beneficial or desired results, including clinical results. Beneficial or desired clinical results can include, but are not limited to, alleviation or amelioration of one or more symptoms or conditions, diminishment of extent of disease, stabilized (i. e. not worsening) state of disease, preventing spread of disease, delay or slowing of disease progression, amelioration or palliation of the disease state, and remission (whether partial or total), whether detectable or undetectable. The term can also mean prolonging survival as compared to expected survival if not receiving treatment.

According to the Global Initiative for Asthma (GINA), 'severe persistent asthma' is defined as a form characterized by daily symptoms, frequent exacerbations, frequent nocturnal asthma symptoms, limitation of physical activities, forced expiratory volume in one second ($FEV_1$) equal to or less than 60% predicted and with a variability higher than 30%.

According to the Global initiative for chronic Obstructive Pulmonary Disease (GOLD) guidelines, 'severe COPD' is a form characterized by a ratio between $FEV_1$ and the Forced Vital Capacity (FVC) lower than 0.7 and $FEV_1$ between 30% and 50% predicted. The very severe form is further characterized by chronic respiratory failure.

The expression 'single therapeutically effective dose' means the quantity of active ingredient administered at one time by inhalation upon actuation of the inhaler. Said dose may be delivered in one or more actuations, preferably one actuation (shot) of the inhaler.

'Actuation' refers to the release of active ingredients from the device by a single activation (e.g. mechanical or breath).

The expression 'water insoluble or poorly water soluble' are used with reference to the solubility in water as defined in the European Pharmacopoeia Ed. 4th, 2003, page 2891.

The term 'UPLC-PDA' refers to a Ultra Performance Liquid Chromatography instrument coupled with a Photodiode Array detector.

**FIGURE**

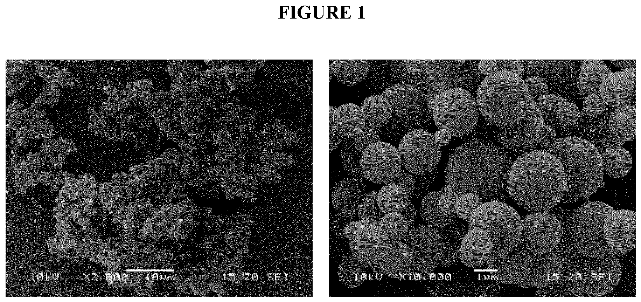

Figure 1 - SEM micrographs of the microparticles of the invention obtained by spray-drying.

## DETAILED DESCRIPTION OF THE INVENTION

The invention is directed to multicomponent microparticles for use in a formulation for inhalation, each microparticle comprising a combination of beclometasone dipropionate, formoterol fumarate, and glycopyrronium bromide.

Formoterol may be present in form of dihydrate fumarate salt.

Glycopyrronium may be used in the form of any of the pure enantiomers or diastereoisomers or any combination thereof in practicing the present invention. In a preferred embodiment, the (3S,2'R), (3R,2'S) 1:1 racemic mixture is used, also known as rac-glycopyrronium.

Beclometasone dipropionate may be anhydrous or present in form of monohydrate.

In a preferred embodiment of the invention, each microparticle consists of a combination of formoterol fumarate or its dihydrate form thereof, glycopyrronium bromide and beclometasone dipropionate.

The ratio by weight in which the three active ingredients, e.g. beclometasone dipropionate, formoterol fumarate, and glycopyrronium bromide, are present in the microparticles is pre-determined in such a way as to deliver the desired single therapeutically effective dose of each active ingredient.

As follows, the ratio by weight among the three active ingredients is given by making reference to the anhydrous form of beclometasone dipropionate (BDP), to the dihydrate fumarate salt of formoterol (FF), and to the bromide salt of glycopyrronium (GB).

Advantageously said ratio is comprised between 35:10:55 and 94:1:5 w/w/w. In a preferred embodiment the ratio could be comprised between 70:10:20 to 92:2:6.

Examples of ratios according to the invention are: 84.4:5.1:10.5 w/w/w to deliver 100 microg BDP, 6 microg FF, and 12.5 microg GB; 91.5:2.7:5.7 w/w/w to deliver 200 microg BDP, 6 microg FF, and 12.5 GB; 73.0:8.8:18.2 w/w/w to deliver 50 microg BDP, 6 microg FF, and 12.5 GB; 80.3: 9.6: 10.1 w/w/w to deliver 100 microg BDP, 12 microg FF, and 12.5 microg GB; 89.1: 5.3:5.6 w/w/w to deliver 200 microg BDP, 12 microg FF, and 12.5 microg GB; 72.7:4.4:22.9 to deliver 200 microg BDP, 12 microg FF and 63 microg GB; 59.2:3.5:37.3 w/w/w to deliver 100 microg BDP, 6 microg FF, and 63 microg GB, 76.3:4.6:19.1 w/w/w to deliver 100 microg BDP, 6 microg FF, and 25 microg, GB and 84.4:5.1:10.5 w/w/w to deliver 200 microg BDP, 12 microg FF, and 25 microg GB.

In a preferred embodiment, the three active ingredients could be present in a ratio of 84.4:5.1:10.5, or 91.5:2.7:5.7 or 73.0:8.8:18.2 w/w/w, more preferably of 84.4:5.1:10.5 w/w/w.

Upon preparation, the microparticles of the invention turned out be chemically stable.

Upon their delivery, they exhibit a good constancy of the active ingredients ratio and give rise high fraction of extrafine particles indicating that could be suitable for the prevention and/or treatment of the diseases affecting the distal tract of the respiratory tree.

Furthermore, the microparticles of the invention have a uniform and regular spherical shape exhibiting more homogeneous forces of adhesion along the whole powder which are in turn associated with the improved DPI performances.

The shape factor is used to characterize the shape of the microparticles.

Accordingly, the microparticles of the invention are characterized by a shape factor comprised between 0.95 and 1.05.

The shape factor is determined according to the following equation reported in Kumar S et al Curr Appl. Phys. Influence of metal powder shape on drag coefficient in a spray jet, 2009, 9, 678-682

$$ \mathrm{SF} = 1/\mathrm{RN} $$

wherein:

RN indicates the roundness of the particle and is calculated by applying the following formula:

$$ \mathrm{RN} = \mathrm{p}^2/4\pi\mathrm{A} $$

wherein p and A are the mean perimeter and area values, respectively, of at least ten spherical particles as measured from Scanning electron microscopy (SEM) images.

Alternatively, the mean perimeter and area may be measured by an optical microscope.

In Kumar S et al it is reported that the shape factors (SF) of circle is 1.00. It is also reported that deviation from unity leads to irregularity of the particle, but particles with a SF value higher than 0.8 can be considered having a spherical shape.

Scanning electron microscopy (SEM) or optical microscopy may also be used to qualitatively appreciate the characteristics of the powder particles of the invention such as particles shape and their surface morphology.

Since the microparticles of the invention should be administered to the lungs by inhalation, at least 90% of them should have a volume diameter equal to or lower than 6 micron.

On the other hand, it is well known that most of the available therapeutic approaches tend to be associated with a poor therapeutic control of individuals exhibiting respiratory diseases affecting the small airways such as the small airways asthma phenotype.

By means of microparticles of the invention, all the three active ingredients could simultaneously reach the distal tract of the respiratory tree whereby they could act synergistically and improve small airways outcomes and associated control.

In fact, said microparticles are characterized by a selected, narrow, and well defined particle size distribution wherein the at least 90% of all of them have a volume diameter lower than 4.5 micron, preferably equal to or lower than 4.0 micron, and their volume median diameter is comprised between 1.0 and 3.0 micron, more advantageously between 1.2 and 2.5 micron, preferably between 1.5 and 2.2 micron.

Advantageously, no more than 10% of said microparticles have a volume diameter lower than 0.2 micron, preferably equal to or lower than 0.5 micron, more preferably equal to or lower than 0.6 micron.

It follows that the width of the particle size distribution of the particles of each active ingredient, expressed as a span, should be advantageously comprised between 1.0 and 4.0, more advantageously between 1.2 and 3.5, preferably between 1.5 and 2.0. According the Chew et al J Pharm Pharmaceut Sci 2002, 5, 162-168, the span corresponds to [d (v, 0.9) - d(v,0.1)]/d(v,0.5).

The size of the particles active ingredient is determined by measuring the characteristic equivalent sphere diameter, known as volume diameter, by laser diffraction. In the reported examples, the volume diameter has been determined using a Malvern apparatus. However, other equivalent apparatus may be used by the skilled person in the art.

When determined by the Brunauer-Emmett-Teller (BET) nitrogen adsorption method according to a procedure known to the skilled person in the art, the Specific Surface Area of the microparticles of the invention shall be comprised between 1.5 and 3.5 $m^2/g$, preferably between 2.0 and 3.0 $m^2/g$, preferably between 2.2 and 2.8 $m^2/g$.

The cohesion and adhesion forces of the microparticles of the invention were also assessed by atomic force microscopy (AFM) according to the experimental procedure reported in Example 2. A bed of alpha-lactose monohydrate powder was used to perform the measurements. This powder bed represents a heterogeneous surface in which the contact area for adhesion is highly variable. The contact area in turn is a dominant factor in determining the force of interaction. Comparison of the obtained adhesion and cohesion forces suggests that there is no statistical difference in the magnitude of the interaction of the microparticles of the invention with themselves or with the lactose powder. In sharp contrast, common micronised active ingredients are either strongly adhesive or cohesive. In virtue of said properties, the microparticles of the invention may exhibit improved dispersion when formulated as interactive ordered mixtures with excipient particles of lactose as a carrier. In fact, they would be less prone to give rise to the formation of stable agglomerates like common micronised active ingredients.

In a further aspect the present invention provides a process for the production of the microparticles of the invention comprising the steps of:

a) preparing a solution of the three active ingredients in a pre-determined ratio in a suitable solvent;
b) generating an aerosol from the solution of said three active ingredients;
c) drying the atomized droplets to yield the microparticles; and
d) isolating the produced microparticles.

As a result of said process, the microparticles are obtained as a completely amorphous powder in that each active ingredient is present in an amorphous form.

As far as the step a) is concerned, the choice of the solvent is critical as, besides having a high solubilising capacity for the three active ingredients, it should have a suitable degree of volatility and diffusion characteristics within the atomized droplets. These properties indeed significantly affect the particle size distribution of the resulting microparticles.

The skilled person in the art shall be able of choosing the solvent in relation to the desired particle size distribution.

Advantageously, the solvent could be selected from the group consisting of methanol, ethanol, water, DMSO, acetonitrile and mixtures thereof.

In a preferred embodiment of the process according to the invention the solvent is a mixture of ethanol: water ranging from 85:15 to 95:5 v/v, preferably 90:10 v/v.

For step b), any aerosol based atomisation system could be used for generation of the aerosol. Various systems for generating aerosols are well-known. The aerosol may, for example, be generated from the desired substance dissolved in a suitable solvent by electrohydrodynamic spraying, high air pressure atomiser or other aerosol generators including pneumatic systems, rotary (spinning-top) systems, spray nozzles, nebulizers, propellant evaporation systems, piezo-lectric transducers and ultrasonic transducers.

In a preferred embodiment, the microparticles are prepared by spray-drying. In this case the solution of step a) is introduced into the drying chamber of a spray-drier through an atomizing device to form droplets and the atomized droplets are dried by introducing a stream of pre-heated drying gas into said drying chamber.

Any commercially available spray-drier may be advantageously used.

The skilled person in the art shall properly adjust the conditions of the aerosol generation such as the temperature of the solution, the solution flow rate and the pressure of the carrier gas in relation with the desired particle size distribution of the microparticles and the size of the batch.

In a particular embodiment of the invention, it might be preferred to obtain partially amorphous or crystalline microparticles in that at least one of the active ingredients is in crystalline form.

In this case, in step d) of the aforementioned process of preparation, the microparticles are collected in a vessel containing an anti-solvent for all the three active ingredients; then a high intensity ultrasound is applied to change the morphology of the microparticles and induce the crystallization of at least one of the three active ingredients present in the microparticle. Finally the microparticles are isolated according to methods known to the skilled person in the art.

The anti-solvent may advantageously be selected from the group consisting of n-heptane, cyclohexane, and fluorinated hydrocarbons such as perfluorodecalin.

Further details about the conditions to induce crystallization are disclosed in WO 2010/007447 and WO 2010/097188.

The partially amorphous or crystalline microparticles could be further isolated and collected.

When the microparticles of the invention comprising formoterol fumarate, beclometasone dipropionate and glycopyrronium bromide are isolated as an amorphous powder, they contain all the three active ingredients in the anhydrous form.

On the contrary, when said microparticles are isolated as partially amorphous or crystalline powder, they might contain formoterol fumarate as dihydrate form and beclometasone dipropionate as monohydrate form.

In a particular embodiment, all the three active ingredient in the microparticles are in a crystalline form.

In this case, advantageously the extent of crystallinity, expressed as weight % of the crystalline microparticle with respect to the total weight of the microparticle, is higher than 90%, preferably higher than 95%.

The amorphicity and/or crystallinity and extent thereof may be determined using X-ray powder diffraction or other techniques known to the skilled person such as differential scanning calorimetry (DSC) or microcalorimetry.

The presence of all the active ingredients in the microparticles could be detected by methods known to the skilled person such as Raman spectroscopy and solid state CP-MAS $^{13}$C NMR spectroscopy.

The microparticles of the invention are physically stable upon storage for at least one month.

However, in the case of amorphous or partially amorphous microparticles, further stabilisation of the amorphous state can be achieved with the aid of suitable excipients in the relevant pharmaceutical formulation for inhalation, for example mannitol, leucine, or trehalose.

Accordingly, in another aspect, the present invention provides a pharmaceutical formulation for administration by inhalation comprising the microparticles of the invention. Said microparticles may be formulated together with one or more pharmaceutically acceptable excipients, additives, diluents or carriers.

For example, the formulation may be provided in the form of suspension in a propellant as aerosol carrier to be administered by pressurized metered dose inhalers (pMDI).

The pMDI comprises a canister wherein the formulation is filled and a metering valve for delivering a daily therapeutically effective dose of the formulation.

In certain embodiments the aerosol carrier may consist of a non-chlorofluorocarbon-based propellant such as hydrofluoralkane (HFA). In particular the propellants HFA 134a, and HFA 227 or mixtures thereof may be advantageously used.

The suspension formulation may comprise additional excipients such as surfactants, and wetting agents.

In a preferred embodiment, the formulation is provided in the form of dry powder for inhalation, more preferably in the form of an interactive or ordered mixture, by diluting the particles of the invention in a pharmacologically inert physiologically acceptable excipient consisting of coarse particles.

Advantageously, said powder formulation for inhalation may comprise the particles according to the invention and coarse particles of a physiologically acceptable excipient, e.g. particles having a MMD higher than 90 micron and preferably the MD comprised between 50 micron and 500 micron, more preferably between 150 and 400 micron, even more preferably between 210 and 355 micron. In another embodiment, the coarse particles have a MD comprised between 90 and 150 micron.

In one of the preferred embodiment, when their MD is comprised between 210 and 355 micron, the coarse excipient particles have preferably a relatively highly fissured surface.

Preferably the aforementioned powder formulation may further comprise a fraction of particles having a MMD lower than 35 micron preferably lower than 15 micron, more preferably lower than 10 micron, composed of particles of a physiologically acceptable excipient and particles of an additive material selected from the class of the anti-adherents such as the amino acids leucine and isoleucine or of the lubricants such as magnesium stearate, sodium stearyl fumarate stearyl alcohol, stearic acid and sucrose monopalmitate (hereinafter the pre-blend fraction).

The physiologically acceptable excipient may be constituted of any amorphous or crystalline physiologically acceptable pharmacologically-inert material of animal or vegetal source or combination thereof. Preferred materials are crystalline sugars and for example monosaccharides such as glucose or arabinose, or disaccharides such as maltose, trehalose, saccharose, dextrose or lactose. Polyalcohols such as mannitol, sorbitol, maltitol, lactitol may also be used. The most preferred material is $\alpha$-lactose monohydrate.

Examples of commercial lactose are Capsulac™, Inhalac™ and Pharmatose™. An example of commercial mannitol is Pearlitol™.

In a preferred embodiment, the pre-blend fraction is composed of 98% by weight of $\alpha$-lactose monohydrate and 2% by weight of magnesium stearate and the ratio between the fraction of microparticles and the fraction of coarse particles made of $\alpha$-lactose monohydrate particles is 10:90% by weight, respectively.

The amount of magnesium stearate in the final formulation is advantageously comprised between 0.02% and 1.0% by weight on the total weight of the formulation, preferably between 0.05 and 0.5% by weight, more preferably between 0.1 and 0.4% by weight, even more preferably between 0.2 and 0.3% by weight.

The powder formulation for inhalation comprising the microparticles according to the invention is endowed with good flowability properties and is characterized by a high degree of homogeneity. In fact, after the mixing, the content uniformity of the active ingredient, expressed as relative standard deviation (RSD), is less than 5%.

Said powder formulation may be administered by inhalation with any type of DPIs known in the art.

DPIs can be divided into two basic types: i) single dose inhalers, for the administration of pre-subdivided single doses of the active compound; ii) multidose dry powder inhalers (MDPIs), either with pre-subdivided single doses or pre-loaded with quantities of active ingredient sufficient for multiple doses. On the basis of the required inspiratory flow rates (l/min) which in turn are strictly depending on their design and mechanical features, DPIs are divided in: i) low-resistance devices (> 90 l/min); ii) medium-resistance devices (about 60 l/min); iii) high-resistance devices (about 30 l/min).

The dry powder formulations comprising the microparticles of the invention are particularly suitable for multidose DPIs comprising a reservoir from which individual therapeutic dosages can be withdrawn on demand through actuation of the device, for example that described in WO 2004/012801.

Other multidose devices that may be used are, for instance, the DISKUS™ of GlaxoSmithKline, the TURBOHALER™ of AstraZeneca, the TWISTHALER™ of Schering and the CLICKHALER™ of Innovata.

As marketed examples of single dose devices, there may be mentioned ROTOHALER™ of GlaxoSmithKline, HANDIHALER™ of Boehringer Ingelheim, Breezehaler™ of Novartis, and Monodose RS01 of Plastiape.

In a preferred embodiment, the dry powder formulation is filled in the DPI device disclosed in WO 2004/012801, being particularly suitable for the delivery of extrafine formulation.

To protect the DPIs from ingress of moisture into the formulation, it may be desirable to overwrap the device in a flexible package capable of resisting moisture ingress such as that disclosed in EP 1760008.

The microparticles of the invention are indicated for the prevention and/or treatment of inflammatory or obstructive airways diseases such as asthma and chronic obstructive pulmonary disease (COPD).

Other respiratory disorders characterised by obstruction of the peripheral airways as a result of inflammation and/or presence of mucus such as chronic obstructive bronchiolitis, bronchiectasies, and cystic fibrosis may also benefit by their use.

In certain embodiments, the microparticles of the invention are particularly suitable for the prevention and/or treatment of severe and/or very severe forms of respiratory disorders, in particular severe and/or very severe COPD and severe persistent asthma.

The invention is further illustrated by the following examples.

## EXAMPLES

### Example 1 - **Manufacture of the microparticles according to the invention**

15 g of a mixture of formoterol fumarate dihydrate (FF), beclometasone dipropionate (BDP) and glycopyrronium bromide (GB) in a 84.4:10.5:5.1% weight ratio was dissolved in 1500 ml of 90:10% v/v ethanol:water.

The feedstock solution was spray dried using a B290 bench top spray dryer (Buchi) with a 2-fluid nozzle at a feed rate of 5g min$^{-1}$.

The spray drying parameters are reported in Table 1.

**Table 1**

| Parameter | Setting |
|---|---|
| Inlet temperature (°C) | 125 |
| Outlet temperature (°C) | 83 |
| Atomisation air pressure (mm) | 35 ($\sim$414 L h$^{-1}$) |
| Aspirator setting (%) | 90 ($\sim$30-35 m$^3$ h$^{-1}$) |
| Filter pressure (mbar) | 30 |

After completion of the spray drying, the cyclone and collection vessel were separated from the B290 spray dryer and stored for 24 hours at a temperature of 25°C and at a relative humidity of 20%.

A flowable powder was recovered with a yield of 77% (11.7 g).

**Example 2** - **Characterization of the microparticles of the invention**

The microparticles as obtained in Example 1 were characterized in terms of drug content, particle size, morphology, physical state, cohesion/adhesion forces and specific surface area.

*Drug content*

It was determined by UPLC-PDA assay (mean $\pm$SD; n=5).

The results are reported in Table 2, indicating that it is within $\pm$3% of the target concentration for each active ingredient (API) in the spray dried powder.

**Table 2**

| API | Actual concentration (%w/w) | Theoretical concentration (% w/w) |
|---|---|---|
| BDP | 82.0 $\pm$ 0.3 | 84.4 |
| GB | 10.6 $\pm$ 0.1 | 10.5 |
| FF | 5.1 $\pm$ 0.0 | 5.1 |

*Particle size*

The particle size was determined by laser diffraction using an R1 lens (Sympatec HELOS). A sample of the powder was dispersed for two measurement conditions using an air pressure of 0.5 and 3 bar respectively (Sympatec RODOS) and sampled at a rate of 5m/s from a controlled temperature and humidity dosing unit (Sympatec ASPIROS). The average d[v,10], d[v,50], d[v,90] values were calculated from triplicate measurements.

The span was calculated using the following equation:

$$\text{Span} = [\text{d}(v, 0.9) - \text{d}(v, 0.1)]/\text{d}(v, 0.5)$$

The values obtained for particle size, which are reported in Table 3, were not affected by the dispersion pressure, indicating a free flowing powder with no hard aggregates.

**Table 3**

| Dispersion pressure (bar) | d[v,10] | d[v,50] | d[v,90] | Span |
|---|---|---|---|---|
| 0.5 | 0.20 ($\pm$0.01) | 1.83 ($\pm$0.06) | 3.72 ($\pm$0.07) | 1.92 |
| 3.0 | 0.20 ($\pm$0.01) | 1.76 ($\pm$0.05) | 3.50 ($\pm$0.04) | 1.88 |

*Morphology*

The morphology of the microparticles was determined by scanning electron microscope (SEM) using Jeol JSM-6480LV instrument.

Samples were mounted on carbon tape and stored in a vacuum for 12 hours prior to analysis to prevent outgassing. Each sample was sputter coated with gold before imaging.

The shape factor analysis was performed as follows. A sample of spray dried powder was dispersed on a 10mm circular glass coverslip and coated with gold using an Agar sputter coater. SEM micrographs were obtained using a LEO1430VP instrument at an accelerating voltage of 10 kV and working distance of 10 mm. 120 SEM micrographs were acquired at 0° tilt and analyzed using Image Pro Analyser Version 7.0. The measurement parameters were set to acquire individual particles for shape analysis and any sampling anomalies i.e. selection of multiple particles, were removed manually. The roundness factor (RN) was determined using the equation $RN = p2/4\pi A$, where p is the perimeter and A is the area. Particle Shape factor (PSF) was calculated from the roundness factor using the equation $PSF = 1/RN$. Statistical analysis was performed on the RN and PSF data to determine whether sufficient particles had been analyzed to give consistent data. The powder exhibited a uniform spherical morphology as demonstrated by SEM pictures (Figure 1). Both the roundness (RN) and particle shape factor determined by image analysis of 3953 individual particles confirmed the spherical morphology of the particles (see Table 4).

**Table 4**

|  | Roundness | Particle shape factor |
|---|---|---|
| Mean | 1.008 | 0.993 |
| S.D. | 0.039 | 0.028 |
| Median | 1.000 | 1.000 |
| N | 3953 | 3953 |

*Physical state*

It was investigated by X-ray powder diffraction (XRPD) and Differential scanning calorimetry (DSC)

XRPD was performed using a Bruker AXS D8 Advance, equipped with a Vantec-1 detector and using Cu K-alpha radiation (1.54 A).

As for DSC, the heat flow (W g$^{-1}$) as a function of increasing temperature (°C) was determined using a TA Instruments Q2000 calorimeter. Samples were weighed into a non-hermetic aluminium DSC pan. The samples were equilibrated at 20°C before the temperature was ramped 5°C min$^{-1}$ up to 220°C. The analysis was performed in triplicate and interpreted using Universal Analysis software.

Both DSC and XRPD indicate that the material is amorphous. In fact, compared to the reference materials, the spray dried microparticles exhibited no peaks corresponding to short range order and crystalline structure and a typical amorphous halo is seen.

No melting point was identifiable in the DSC trace.

*Cohesion/adhesion forces*

The force measuring capabilities of the atomic force microscope (AFM) were used to directly measure the adhesion forces between two surfaces.

Tipless contact mode AFM cantilevers (Bruker) with calibrated spring constant (typically 0.2-0.4 Nm$^{-1}$) were used for all adhesion-cohesion measurements. Probes were prepared by attaching the microparticles to the tip of the cantilever, which was confirmed by optical and variable pressure SEM before use. A bed of the spray dried microparticles or Inhalac 50 lactose was fixed onto a silicon substrate using a thin layer of glue. Force-distance curves were recorded by monitoring the deflections of the cantilever as the probe and sample were brought into contact (approach trace), and then separated (retract trace). Cohesion/adhesion data was obtained from the microparticles and lactose samples consecutively with the same tip. Data was obtained in a 5x5 grid with 500nm spacing. Each grid generates 25 force curves over a $2 \times 2\mu m$ area. The experiment was repeated with a different particle probe or different sampling area. All experiments were conducted at ambient environmental conditions. A total of 153 cohesion and 144 adhesion force curves were acquired and processed to extract the interaction force (nN).

Comparison of the obtained adhesion and cohesion forces suggests that there is no statistical difference in the magnitude of the interaction of the microparticles of the invention with themselves or with the lactose powder. In sharp contrast, common micronised active ingredients are either strongly adhesive or cohesive. In virtue of said properties, the microparticles of the invention may exhibit improved dispersion when formulated as interactive ordered mixtures with excipient particles of lactose as a carrier. In fact, they would be less prone to give rise to the formation of stable agglomerates like common micronised active ingredients.

*Specific surface area*

The specific surface area was determined using nitrogen multipoint BET (Brunauer-Emmet-Teuer) with a TriStar II 3020 instrument.

The values are reported in Table 5.

**Table 5**

|  | BET surface area (m$^2$ g$^{-1}$) | Total volume of pores < 3063.514 Å (cm$^3$ g$^{-1}$) | BJH adsorption average pore diameter (Å) |
|---|---|---|---|
| Run 1 | 2.6120 | 0.004248 | 6.0434 |
| Run 2 | 2.3339 | 0.004556 | 5.9286 |
| Run 3 | 2.5183 | 0.004104 | 6.1602 |
| Mean | 2.488 | 0.004 | 6.044 |
| S.D. | 0.14 | 0.00 | 0.12 |

**Example 3** - **Dry powder formulation comprising the microparticles of the invention**

A hard pellet carrier containing coarse lactose (sieve fraction 212-355 $\mu$m) and co-micronised pre-blend in a ratio of 9:1 was prepared before manufacture of the formulation according to the teaching of WO 01/78693. Briefly, alpha-lactose monohydrate particles (sieve fraction 212-355 $\mu$m) and a pre-blend fraction of alpha-lactose monohydrate and magnesium stearate were mixed in a ratio of 9:1.

A powder formulation (50 g batch size) containing 1.22% w/w of the microparticles of Example 1 equivalent to 100 $\mu$g BDP, 12.5 $\mu$g GB, and 6.0 $\mu$g FF in 10 mg dose, was prepared in a Turbula mixer. It was prepared by separately weighing out the hard pellet carrier and the microparticles. Half the carrier was added to a stainless steel vessel followed by the microparticles. The remaining carrier was added and the contents mixed at 32 rpm for 90 minutes. The formulation was then sieved using a 500 $\mu$m sieve and mixed for a further 30 minutes at 32 rpm. The formulation was stored in an amber glass jar for a minimum of 24 hours at 20°C an 40% relative humidity prior to further analysis.

**Example 4**

The powder formulation of Example 3 was characterized in terms of the uniformity of distribution of the active ingredients and aerosol performances after loading it in the multidose dry powder inhaler described in WO 2004/012801, and quoted hereinafter as NEXThaler®.

*Uniformity of distribution*

About 10 mg of the formulation was weighed directly into a 25 mL volumetric flask. It was sampled 10 times. The flask was made up to volume with 60:40% v/v MeOH:H$_2$O and sonicated for 2 minutes. The samples were analysed for each active ingredient using UPLC-PDA assay.

The results (mean value $\pm$ RSD) are reported in Table 6.

**Table 6**

| Drug | Content ($\mu$g 10 mg$^{-1}$) | RSD (%) | % of Target |
|---|---|---|---|
| BDP | 98.75 $\pm$ 1.23 | 1.25 | 98.75 |
| GB | 12.64 $\pm$ 0.18 | 1.45 | 105.37 |
| FF | 6.08 $\pm$ 0.08 | 1.24 | 101.27 |

*Aerosol performances*

Aerosol performances were determined using the Next Generation Impactor (NGI) with a USP induction port and pre-separator containing 15mL 60:40% v/v MeOH:H$_2$O. Critical flow (P$_3$/P$_2$ ratio) was <0.5 at the sampling flow rate of 60 L/min. The aerodynamic particle size distribution was based on 5 actuations from the NEXThaler, each sampled into 4 liters of air (equivalent to an inhalation time of 4 s). The device was weighed before and after each actuation to determine the shot weight (mg). A minimum of 1 minute was allowed between consecutive actuations to allow static charge to dissipate.

Samples were collected from the NGI using a fixed volume technique. MeOH:H₂O (60:40% v/v) was dispensed into the induction port (including mouthpiece) ; pre-separator; and each stage using an electronic metering dispenser. The induction port (30mL) and pre-separator (50mL) were sealed using silicone bungs and shaken by hand for 2 minutes before the samples were collected. The NGI stages (10mL stages 1-2; 15mL stage 3-MOC) were rocked using a NGI rocker for 3 minutes. All samples were filtered using a 0.2$\mu$m syringe filter before analysis by UPLC-PDA.

The following parameters, were calculated: i) the delivered dose (DD) which is the amount of drug delivered from the device recovered in the all parts of impactor; ii) the fine particle mass (FPM) which is the amount of delivered dose having a particle size equal to or lower than 5.0 micron iii) the fine particle fraction (FPF) which is the percentage of the fine particle dose; iv) the MMAD; and v) the extrafine FPP which is the percentage of the fine particle dose having a particle size equal to or lower than 2.0 micron.

The results (mean value $\pm$ S.D, n= 6) are reported in Table 7.

**Table 7**

| Drug | DD ($\mu$g) | FPM ($\mu$g) | FPF (%) | MMAD ($\mu$m) | FPF < 1$\mu$m | FPF < 2$\mu$m |
|------|-------------|--------------|---------|---------------|---------------|---------------|
| FF | 5.2 $\pm$0.1 | 3.7 $\pm$0.2 | 70.2 $\pm$3.0 | 1.9 $\pm$0.1 | 21.6$\pm$1.0 | 39.7$\pm$1.1 |
| GB | 11.5 $\pm$0.4 | 7.9 $\pm$0.5 | 68.5 $\pm$3.7 | 1.9 $\pm$0.1 | 21.7$\pm$0.7 | 39.3$\pm$1.6 |
| BDP | 87.6 $\pm$2.2 | 60.0 $\pm$3.6 | 68.5 $\pm$3.1 | 1.9 $\pm$0.1 | 20.8$\pm$0.7 | 38.6$\pm$1.2 |

From the data of Tables 6 and 7, it can be appreciated that the prepared formulation shows both an excellent homogeneity with RSD values < 2% and high delivered dose and respirable fraction (FPF), for all the three active ingredients.

They also give rise to a high fraction of particles having a diameter equal or less than 2 micron (at least more than 35% for all the active ingredients), indicating that could be suitable for the prevention and/or treatment of the diseases affecting the distal tract of the respiratory tree.

**Claims**

1. Multicomponent microparticles for use in a formulation for inhalation, each microparticle comprising a combination of beclometasone dipropionate, formoterol fumarate, and glycopyrronium bromide, as active ingredients, in a ratio comprised between 35:10:55 and 94:1:5 w/w/w, whereby said microparticles are **characterized by** a shape factor comprised between 0.95 and 1.05 determined according to the following equation:

$$SF = 1/RN$$

wherein RN indicates the roundness of the particle and is calculated by applying the following formula:

$$RN = p^2 / 4\pi A$$

wherein p and A are the mean perimeter and area values, respectively, of at least ten spherical particles as measured from Scanning electron microscopy (SEM) images, and
wherein said microparticles are obtained by a process comprising the steps of:

   a) preparing a solution of the three active ingredients in a pre-determined ratio in a solvent consisting of a mixture of ethanol:water in a ratio of from 85:15 to 95:5 v/v;
   b) generating an aerosol from the solution of said three active ingredients; and
   c) drying the atomized droplets to yield the microparticles.

2. The microparticles according to claim 1, wherein at least 90% of all said microparticles have a volume diameter equal to or lower than 4.5 micron, and the volume median diameter of said microparticles is comprised between 1.0 and 3.0 micron.

3. The microparticles according to claim 2, wherein no more than 10% of said microparticles have a volume diameter lower than 0.2 micron.

4. The microparticles according to any one of claims 1 to 3, wherein each microparticle consists of a combination of formoterol fumarate or its dihydrate form thereof, glycopyrronium bromide and beclometasone dipropionate.

5. The microparticles according to any one of claims 1 to 4, wherein each active ingredient is present in an amorphous form.

6. A process for preparing multicomponent microparticles for use in a formulation for inhalation, each microparticle comprising a combination of beclometasone dipropionate, formoterol fumarate, and glycopyrronium bromide, as active ingredients, in a ratio comprised between 35:10:55 and 94:1:5 w/w/w, whereby said microparticles are **characterized by** a shape factor comprised between 0.95 and 1.05 determined according to the following equation:

$$SF = 1/RN$$

wherein RN indicates the roundness of the particle and is calculated by applying the following formula:

$$RN = p^2/4\pi A$$

wherein p and A are the mean perimeter and area values, respectively, of at least ten spherical particles as measured from Scanning electron microscopy (SEM) images,
said process comprising the steps of:

   a) preparing a solution of the three active ingredients in a pre-determined ratio in a suitable solvent;
   b) generating an aerosol from the solution of said three active ingredients;
   c) drying the atomized droplets to yield the microparticles; and
   d) isolating the produced microparticles.

7. The process according to claim 6, wherein the solvent is selected from the group consisting of methanol, ethanol, water, DMSO, acetonitrile and mixtures thereof.

8. The microparticles according to any one of claims 1 to 4, wherein at least one of the active ingredients is in crystalline form.

9. A process for preparing multicomponent microparticles for use in a formulation for inhalation, each microparticle comprising a combination of beclometasone dipropionate, formoterol fumarate, and glycopyrronium bromide, as active ingredients, in a ratio comprised between 35:10:55 and 94:1:5 w/w/w, whereby said microparticles are **characterized by** a shape factor comprised between 0.95 and 1.05 determined according to the following equation:

$$SF = 1/RN$$

wherein RN indicates the roundness of the particle and is calculated by applying the following formula:

$$RN = p^2/4\pi A$$

wherein p and A are the mean perimeter and area values, respectively, of at least ten spherical particles as measured from Scanning electron microscopy (SEM) images, wherein at least one of the active ingredients is in crystalline form, said process comprising the steps of:

   a) preparing a solution of the three active ingredients in a pre-determined ratio in a suitable solvent;
   b) generating an aerosol from the solution of said three active ingredients;
   c) drying the atomized droplets to yield the microparticles

d) collecting the obtained microparticles in a vessel containing an anti-solvent for all the three active ingredients;

e) applying a high intensity ultrasound to change the morphology of the microparticles and induce the crystallization;

f) isolating the produced microparticles.

10. The process according to claim 9, wherein the anti-solvent is selected from the group consisting of n-heptane, cyclohexane, and fluorinated hydrocarbons such as perfluorodecalin.

11. A pharmaceutical aerosol formulation for pressurized metered dose inhalers (pMDIs) comprising the microparticles of any one of claims 1 to 4 in suspension in a liquefied propellant gas.

12. A pressurized metered dose inhaler (pMDI) comprising a canister filled with the pharmaceutical aerosol formulation of claim 11, and a metering valve for delivering a daily therapeutically effective dose of the active ingredient.

13. A dry powder pharmaceutical formulation comprising the microparticles of any one of claims 1 to 4 and, optionally a carrier.

14. A dry powder inhaler filled with the dry powder formulation of claim 13.

15. The microparticles of any one of claims 1 to 4 for use in the prevention and/or treatment of an inflammatory and/or obstructive airways disease.

16. The microparticles for use according to claim 15, wherein the disease is asthma or chronic obstructive pulmonary disease (COPD).

## Patentansprüche

1. Multikomponentenmikropartikel zur Verwendung in einer Formulierung zur Inhalation, wobei jedes Mikropartikel eine Kombination von Beclometasondipropionat, Formoterolfumarat und Glycopyrroniumbromid, als aktive Ingredientien, in einem Verhältnis, das zwischen 35:10:55 und 94:1:5 G/G/G liegt, umfasst, wobei die Mikropartikel durch einen Formfaktor gekennzeichnet sind, der zwischen 0,95 und 1,05 liegt, der nach der folgenden Gleichung bestimmt wurde:

$$SF = 1/RN$$

worin RN die Rundheit des Partikels angibt und durch Anwenden der folgenden Formel errechnet wird:

$$RN = p^2/4\pi A$$

worin p und A die mittleren Umfangs- bzw. Flächenwerte von wenigstens zehn sphärischen Partikeln, wie sie aus Rasterelektronenmikroskopie (REM)-Bildern gemessen worden sind, sind und

wobei die Mikropartikel durch ein Verfahren erhalten werden, das die folgenden Schritte umfasst:

a) Herstellen einer Lösung der drei aktiven Ingredienzien in einem vorbestimmten Verhältnis in einem Lösungsmittel, das aus einem Gemisch von Ethanol:Wasser in einem Verhältnis von 85:15 bis 95:5 V/V besteht;

b) Erzeugen eines Aerosols aus der Lösung der drei aktiven Ingredienzien und

c) Trocknen der atomisierten Tröpfchen unter Erhalt der Mikropartikel.

2. Mikropartikel nach Anspruch 1, wobei wenigstens 90% aller Mikropartikel einen Volumendurchmesser von gleich oder kleiner als 4,5 Mikrometer haben und der volumenmediane Durchmesser der Partikel zwischen 1,0 und 3,0 Mikrometer liegt.

3. Mikropartikel nach Anspruch 2, wobei nicht mehr als 10% der Mikropartikel einen Volumendurchmesser von kleiner als 0,2 Mikrometer haben.

4. Mikropartikel nach einem der Ansprüche 1 bis 3, wobei jedes Mikropartikel aus einer Kombination von Formoterolfumarat oder seiner Dihydratform, Glycopyrroniumbromid und Beclometasondipropionat besteht.

5. Mikropartikel nach einem der Ansprüche 1 bis 4, wobei jedes aktive Ingrediens in einer amorphen Form vorliegt.

6. Verfahren zur Herstellung von Multikomponentenmikropartikeln zur Verwendung in einer Formulierung zur Inhalation, wobei jedes Mikropartikel eine Kombination von Beclometasondipropionat, Formoterolfumarat und Glycopyrroniumbromid, als aktive Ingredientien, in einem Verhältnis, das zwischen 35:10:55 und 94:1:5 G/G/G liegt, umfasst, wobei die Mikropartikel durch einen Formfaktor gekennzeichnet sind, der zwischen 0,95 und 1,05 liegt, der nach der folgenden Gleichung bestimmt wurde:

$$SF = 1/RN$$

worin RN die Rundheit des Partikels angibt und durch Anwenden der folgenden Formel errechnet wird:

$$RN = p^2/4\pi A$$

worin p und A die mittleren Umfangs- bzw. Flächenwerte von wenigstens zehn sphärischen Partikeln, wie sie aus Rasterelektronenmikroskopie (REM)-Bildern gemessen worden sind, sind
wobei das Verfahren die folgenden Schritte umfasst:

  a) Herstellen einer Lösung der drei aktiven Ingredienzien in einem vorbestimmten Verhältnis in einem geeigneten Lösungsmittel;
  b) Erzeugen eines Aerosols aus der Lösung der drei aktiven Ingredienzien;
  c) Trocknen der atomisierten Tröpfchen unter Erhalt der Mikropartikel; und
  d) Isolieren der hergestellten Mikropartikel.

7. Verfahren nach Anspruch 6, wobei das Lösungsmittel aus der Gruppe, bestehend aus Methanol, Ethanol, Wasser, DMSO, Acetonitril und Gemischen davon, ausgewählt wird.

8. Mikropartikel nach einem der Ansprüche 1 bis 4, wobei wenigstens eins der aktiven Ingredienzien in kristalliner Form ist.

9. Verfahren zur Herstellung von Multikomponentenmikropartikeln zur Verwendung in einer Formulierung zur Inhalation, wobei jedes Mikropartikel eine Kombination von Beclometasondipropionat, Formoterolfumarat und Glycopyrroniumbromid, als aktive Ingredientien, in einem Verhältnis, das zwischen 35:10:55 und 94:1:5 G/G/G liegt, umfasst, wobei die Mikropartikel durch einen Formfaktor gekennzeichnet sind, der zwischen 0,95 und 1,05 liegt, der nach der folgenden Gleichung bestimmt wurde:

$$SF = 1/RN$$

worin RN die Rundheit des Partikels angibt und durch Anwenden der folgenden Formel errechnet wird:

$$RN = p^2/4\pi A$$

worin p und A die mittleren Umfangs- bzw. Flächenwerte von wenigstens zehn sphärischen Partikeln, wie sie aus Rasterelektronenmikroskopie (REM)-Bildern gemessen worden sind, sind, wobei wenigstens eins der aktiven Ingredientien in kristalliner Form ist,
wobei das Verfahren die folgenden Schritte umfasst:

  a) Herstellen einer Lösung der drei aktiven Ingredienzien in einem vorbestimmten Verhältnis in einem geeigneten Lösungsmittel;
  b) Erzeugen eines Aerosols aus der Lösung der drei aktiven Ingredienzien;

c) Trocknen der atomisierten Tröpfchen unter Erhalt der Mikropartikel;

d) Sammeln der erhaltenen Mikropartikel in einem Gefäß, das ein Antilösungsmittel für alle drei aktiven Ingredienzien enthält;

e) Anwenden von Ultraschall hoher Intensität, um die Morphologie der Mikropartikel zu verändern und die Kristallisation zu induzieren;

f) Isolieren der hergestellten Mikropartikel.

10. Verfahren nach Anspruch 9, wobei das Antilösungsmittel aus der Gruppe, bestehend aus n-Heptan, Cyclohexan und fluorierten Kohlenwasserstoffen, zum Beispiel Perfluordecalin, ausgewählt wird.

11. Pharmazeutische Aerosolformulierung für Druckdosierinhalatoren (pressurized metered dose inhalers (pMDIs)), umfassend die Mikropartikel nach einem der Ansprüche 1 bis 4 in Suspension in einem verflüssigten Treibgas.

12. Druckdosierinhalator (pMDI), umfassend eine Kartusche, die mit der pharmazeutischen Aerosolformulierung von Anspruch 11 gefüllt ist, und ein Dosierventil zur Abgabe einer täglichen therapeutisch wirksamen Dosis des aktiven Ingrediens.

13. Pharmazeutische Trockenpulverformulierung, umfassend die Mikropartikel nach einem der Ansprüche 1 bis 4 und gegebenenfalls einen Träger.

14. Trockenpulverinhalator, gefüllt mit der Trockenpulverformulierung von Anspruch 13.

15. Mikropartikel nach einem der Ansprüche 1 bis 4 zur Verwendung bei der Prävention und/oder Behandlung einer inflammatorischen und/oder obstruktiven Atemwegserkrankung.

16. Mikropartikel zur Verwendung nach Anspruch 15, wobei die Krankheit Asthma oder chronisch-obstruktive Lungenerkrankung (COPD) ist.


**Revendications**

1. Microparticules du type à plusieurs composants pour leur utilisation dans une formulation pour inhalation, chaque microparticule comprenant une combinaison de dipropionate de béclométasone, de fumarate de formotérol et de bromure de glycopyrronium, à titre d'ingrédients actifs, dans un rapport compris entre 35:10:55 et 94:1:5 en poids/poids/poids ; dans lesquelles lesdites microparticules sont **caractérisées par** un facteur de forme compris entre 0,95 et 1,05 que l'on détermine conformément à l'équation suivante :

$$SF = 1/RN$$

dans laquelle RN désigne la rondeur de la particule et est calculé en appliquant la formule suivante :

$$RN = p^2/4\pi A$$

dans laquelle p et A représentent les valeurs moyennes du périmètre et de la surface, respectivement d'au moins dix particules sphériques, telles qu'on les mesure à partir d'images obtenues via une microscopie électronique à balayage (SEM) ; et

dans lesquelles lesdites microparticules sont obtenues en passant par un procédé comprenant les étapes dans lesquelles :

a) on prépare une solution des trois ingrédients actifs dans un rapport prédéterminé dans un solvant constitué d'un mélange éthanol:eau dans un rapport de 85:15 à 95:5 en volume/volume ;

b) on génère un aérosol à partir de la solution desdits trois ingrédients actifs ; et

c) on sèche les gouttelettes atomisées afin d'obtenir les microparticules.

2. Microparticules selon la revendication 1, dans lesquelles au moins 90 % de la totalité desdites microparticules possèdent un diamètre en volume égal ou inférieur à 4,5 microns et le diamètre médian en volume desdites microparticules est compris entre 1,0 et 3,0 microns.

3. Microparticules selon la revendication 2, dans lesquelles pas plus de 10 % desdites microparticules possèdent un diamètre en volume inférieur à 0,2 micron.

4. Microparticules selon l'une quelconque des revendications 1 à 3, dans lesquelles chaque microparticule est constituée d'une combinaison de fumarate de formotérol ou de sa forme dihydrate, du bromure de glycopyrronium et du dipropionate de béclométasone.

5. Microparticules selon l'une quelconque des revendications 1 à 4, dans lesquelles chaque ingrédient actif est présent sous une forme amorphe.

6. Procédé pour la préparation de microparticules du type à plusieurs composants pour leur utilisation dans une formulation pour inhalation, chaque microparticule comprenant une combinaison de dipropionate de béclométasone, de fumarate de formotérol et de bromure de glycopyrronium, à titre d'ingrédients actifs, dans un rapport compris entre 35:10:55 et 94:1:5 en poids/poids/poids ; dans lequel lesdites microparticules sont **caractérisées par** un facteur de forme compris entre 0,95 et 1,05 que l'on détermine conformément à l'équation suivante :

$$SF = 1/RN$$

dans laquelle RN désigne la rondeur de la particule et est calculé en appliquant la formule suivante :

$$RN = p^2/4\pi A$$

dans laquelle p et A représentent les valeurs moyennes du périmètre et de la surface, respectivement d'au moins dix particules sphériques, telles qu'on les mesure à partir d'images obtenues via une microscopie électronique à balayage (SEM) ;
ledit procédé comprenant les étapes dans lesquelles :

a) on prépare une solution des trois ingrédients actifs dans un rapport prédéterminé dans un solvant approprié ;
b) on génère un aérosol à partir de la solution desdits trois ingrédients actifs ;
c) on sèche les gouttelettes atomisées afin d'obtenir les microparticules ; et
d) on isole les microparticules obtenues.

7. Procédé selon la revendication 6, dans lequel le solvant est choisi parmi le groupe constitué par le méthanol, l'éthanol, l'eau, le DMSO, l'acétonitrile, ainsi que leurs mélanges.

8. Microparticules selon l'une quelconque des revendications 1 à 4, dans lesquelles au moins un des ingrédients actifs est présent sous forme cristalline.

9. Procédé pour la préparation de microparticules du type à plusieurs composants pour leur utilisation dans une formulation pour inhalation, chaque microparticule comprenant une combinaison de dipropionate de béclométasone, de fumarate de formotérol et de bromure de glycopyrronium, à titre d'ingrédients actifs, dans un rapport compris entre 35:10:55 et 94:1:5 en poids/poids/poids ; dans lequel lesdites microparticules sont **caractérisées par** un facteur de forme compris entre 0,95 et 1,05 que l'on détermine conformément à l'équation suivante :

$$SF = 1/RN$$

dans laquelle RN désigne la rondeur de la particule et est calculé en appliquant la formule suivante :

$$RN = p^2/4\pi A$$

dans laquelle p et A représentent les valeurs moyennes du périmètre et de la surface, respectivement d'au moins dix particules sphériques, telles qu'on les mesure à partir d'images obtenues via une microscopie électronique à balayage (SEM), dans lequel au moins un des ingrédients actifs est présent sous forme cristalline ;
ledit procédé comprenant les étapes dans lesquelles :

a) on prépare une solution des trois ingrédients actifs dans un rapport prédéterminé dans un solvant approprié ;
b) on génère un aérosol à partir de la solution desdits trois ingrédients actifs ;
c) on sèche les gouttelettes atomisées afin d'obtenir les microparticules ;
d) on récolte les microparticules obtenues dans un récipient contenant un antisolvant pour la totalité des trois ingrédients actifs ;
e) on applique des ultrasons de haute intensité afin de modifier la morphologie des microparticules et d'induire la cristallisation ;
f) on isole les microparticules obtenues.

**10.** Procédé selon la revendication 9, dans lequel l'antisolvant est choisi parmi le groupe constitué par le n-heptane, le cyclohexane et des hydrocarbures fluorés tels que la perfluorodécaline.

**11.** Formulation d'aérosol pharmaceutique destinée à des aérosols-doseurs mis sous pression (pMDI) comprenant les microparticules selon l'une quelconque des revendications 1 à 4 en suspension dans un gaz propulseur liquéfié.

**12.** Aérosol-doseur mis sous pression (pMDI) comprenant une cartouche remplie avec la formulation d'aérosol pharmaceutique selon la revendication 11, et une valve doseuse pour distribuer une dose quotidienne thérapeutiquement efficace de l'ingrédient actif.

**13.** Formulation pharmaceutique de poudre sèche comprenant les microparticules selon l'une quelconque des revendications 1 à 4 et, de manière facultative, un support.

**14.** Inhalateur de poudre sèche rempli avec la formulation de poudre sèche selon la revendication 13.

**15.** Microparticules selon l'une quelconque des revendications 1 à 4 pour leur utilisation dans la prévention et/ou dans le traitement d'une maladie inflammatoire et/ou obstructive des voies aériennes.

**16.** Microparticules pour leur utilisation selon la revendication 15, dans lesquelles la maladie est l'asthme ou une maladie pulmonaire obstructive chronique (MPOC).

FIGURE 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0228377 A
- WO 2010097188 A
- WO 2013021199 A
- US 2011150782 A
- US 2011262543 A
- WO 0178695 A
- WO 0178693 A
- WO 2010007447 A
- WO 2004012801 A
- EP 1760008 A

### Non-patent literature cited in the description

- European Pharmacopeia (Eur. Ph.) 7.3
- the European Pharmacopeia (Eur. Ph.) 7.3
- the European Pharmacopoeia Ed. 4. 2003, 2891
- **KUMAR S et al.** Influence of metal powder shape on drag coefficient in a spray jet. *Curr Appl. Phys.,* 2009, vol. 9, 678-682
- **CHEW et al.** *J Pharm Pharmaceut Sci,* 2002, vol. 5, 162-168